# FASCICULE DE BREVET EUROPEEN

(11) **EP 0 710 478 B1**
(45) Date de publication et mention de la délivrance du brevet: **22.01.1997**
(21) Numéro de dépôt: 95402173.9
(22) Date de dépôt: 27.09.1995
(51) Int. Cl.: A61K 7/48, A61K 7/06, A61K 47/46

(54) **Produit pour application topique contenant une lipase et un precurseur d'actif**
Produkt zur topischen Anwendung, das eine Lipase und einen Wirkstoffprecursor enthaelt
Composition for topical application containing a lipase and an active agent precursor

(30) Priorité: 24.10.1994 FR 9412684
(43) Date de publication de la demande: 08.05.1996
(73) Titulaire: L'OREAL, 75008 Paris (FR)
(72) Inventeur: de Salvert, Armelle, F-75013 Paris (FR); Sera, Daniel, F-94150 L'Hay-Les-Roses (FR); Guth, Gérard, F-95160 Montmorency (FR); Fodor, Pierre, F-92380 Garches (FR); Maurin, Emmanuelle, F-95290 L'Isle-Adam (FR)
(74) Mandataire: Lhoste, Catherine

(56) Documents cités:
- WO-A-83/03061
- FR-A- 2 211 209
- FR-A- 2 556 218
- PATENT ABSTRACTS OF JAPAN vol. 12, no. 333 (C-526) (3180) & JP-A-63 096 109 (SHISEIDO) 27 Avril 1988
- PATENT ABSTRACTS OF JAPAN vol. 14, no. 294 (C-732) (4237) & JP-A-02 096 510 (KANEBO) 9 Avril 1990

## Description

La présente invention a pour objet un produit pour application topique apte à libérer sur la peau un actif cosmétique et/ou dermatologique, et son utilisation pour le traitement cosmétique et/ou dermatologique de la peau, y compris le cuir chevelu.

Il est connu d'introduire dans les compositions cosmétiques et/ou dermatologiques des actifs en vue d'apporter des traitements spécifiques à la peau, par exemple pour lutter contre le dessèchement, le vieillissement ou la pigmentation de la peau, pour traiter l'acné ou certaines maladies de la peau (eczéma, psoriasis), pour combattre les surcharges pondérales, pour favoriser la restructuration de la peau ou son renouvellement cellulaire, ou aussi pour colorer la peau.

Par exemple, l'acide ascorbique (ou vitamine C) est connu pour stimuler la croissance du tissu conjonctif, et notamment celle du collagène. Il permet aussi de renforcer les défenses du tissu cutané contre les agressions extérieures telles que le rayonnement ultraviolet ou la pollution. Il est également utilisé pour enlever les taches et la pigmentation de la peau, et aussi pour favoriser la cicatrisation de la peau.

Il est connu aussi que l'application de rétinol ou vitamine A permet de lutter notamment contre le vieillissement cutané et contre certains désordres de la peau tels que l'acné ou les troubles de la kératinisation ou de la cicatrisation.

En outre, les tocophérols, tels que la vitamine E, sont connus pour posséder des propriétés antioxydantes vis à vis des phospholipides de la membrane cellulaire et des propriétés antiradicalaires (voir "Radicaux libres et Vitamine E" de J.B. Chazan et M. Szulc - Cah. Nutr. Diet. 1987, 6, XXII, 1, pages 66 à 76).

Par ailleurs, on sait que l'application de la dihydroxyacétone sur la peau permet de conférer à celle-ci l'apparence d'une peau bronzée, sans avoir les inconvénients (brûlures, risques de cancer) rencontrés lors d'une exposition au soleil.

Malheureusement, certains actifs, et en particulier ceux cités ci-dessus, sont instables et sont sensibles à des facteurs extérieurs tels que la lumière ou la chaleur. Cette instabilité va à l'encontre de l'efficacité recherchée et peut, de plus, être source de désagréments pour l'utilisateur, par exemple lorsque l'instabilité de l'actif entraîne des modifications de couleur et/ou d'odeur de la composition le contenant.

Aussi, différents moyens ont été envisagés pour stabiliser ces actifs. Un de ces moyens consiste par exemple à bloquer le site réactif de l'actif par estérification avec notamment des dérivés phosphatés, sulfatés ou alkylés et à utiliser ces dérivés à la place de l'actif libre. Malheureusement, ces dérivés présentent une efficacité moins bonne que l'actif libre.

Il a été aussi envisagé d'utiliser des précurseurs de tels actifs, qui, après application sur la peau, sont coupés par les enzymes cutanées et libèrent alors l'actif libre. Ainsi, le document EP-A-487404 divulgue l'utilisation d'un dérivé glucosylé de l'acide ascorbique, dans des compositions dermatologiques, facilement hydrolysé par les enzymes cutanées et donc apte à libérer de l'acide ascorbique lorsque ces compositions sont appliquées sur la peau. Mais l'utilisation de tels dérivés ne permet pas une libération rapide et en quantité suffisante d'acide ascorbique à la surface de la peau.

Il subsiste donc le besoin d'un produit pour application topique contenant ces actifs cosmétiques et'ou dermatologiques, dans lequel ces derniers conservent toutes leurs propriétés et donc leur efficacité au cours du temps.

La demanderesse a maintenant trouvé de manière inattendue que l'utilisation d'une enzyme particulière, la lipase, associé à des esters d'actifs instables, permettait d'éviter les inconvénients de l'art antérieur.

Aussi, la présente invention a pour objet un produit pour application topique contenant une enzyme et au moins un précurseur d'un actif cosmétique et/ou dermatologique comportant au moins une fonction hydroxyle, caractérisé en ce que l'enzyme est une lipase, en ce que l'actif cosmétique et/ou dermatologique est choisi dans le groupe comprenant les vitamines estérifiables et les cétones hydroxylées et en ce que le précurseur est un ester comportant au moins une fonction ester à chaîne linéaire ou ramifiée, saturée ou insaturée, ayant de 2 à 25 atomes de carbone.

Par produit, on entend aussi bien la partie cosmétique ou dermatologique (ensemble de la composition) que le dispositif commercial comportant un récipient dans lequel sont logés l'enzyme et le précurseur isolés l'un de l'autre.

La lipase est une enzyme connue pour hydrolyser les triglycérides en mono- et diglycérides, en glycérol et en acides gras libres. Elle est en particulier utilisée dans les détergents (voir l'article "Lipases as detergent components", H. Andree et al., Journal of Applied Biochemistry, 1980, vol. 2, pages 218 à 229) afin de permettre l'élimination des taches graisseuses telles que celles provenant des matières grasses de friture, d'huiles, de sebum ou de cosmétiques gras comme les rouges à lèvres. Du fait de sa propriété de couper les triglycérides, elle a été utilisée dans le domaine cosmétique sous forme immobilisée pour nettoyer la peau (voir par exemple US-A-4556554).

La lipase selon l'invention doit être suffisamment stable pour conserver son activité enzymatique. Elle appartient au groupe des enzymes de classification EC 3.1.1.3., ce qui correspond à une lipase qui coupe les liaisons ester en positions 1 et 3 d'un triglycéride. Elle peut être choisie par exemple parmi celles vendues sous les dénominations commerciales "lipase SP644" et "lipolase 100 L" par la société Novo Nordisk.

La lipase peut être utilisée dans le produit selon l'invention en une quantité allant de 0,05 % à 30 % en poids, de préférence de 0,1 à 10 % en poids par rapport au poids total de la composition, et mieux de 0,1 à 5 % en poids par rapport au poids total de la composition.

Les actifs auxquels s'applique l'invention sont ceux comportant au moins une fonction hydroxyle, et notamment les vitamines estérifiables telles que le rétinol (vitamine A) et ses dérivés, l'acide ascorbique (vitamine C) et ses dérivés ; les cétones hydroxylées telles que la dihydroxyacétone.

L'ester utilisé selon l'invention est un ester comportant une ou plusieurs fonctions ester à chaîne linéaire ou ramifiée, saturée ou insaturée, ayant de 2 à 25 atomes de carbone, comportant éventuellement un ou plusieurs substituants. La chaîne de la fonction ester est choisie en particulier parmi les radicaux acyle, benzoyle, alkylbenzoyle, acylbenzoyle, 2-hydroxyphénylacétyle, éventuellement substitués. Le substituant peut être en particulier un radical hydroxylé.

Selon une réalisation préférée de l'invention, la chaîne de la fonction ester a de 12 à 18 atomes de carbone.

Il s'agit par exemple d'un ester choisi parmi les esters d'acide laurique, d'acide palmitique, d'acide stéarique, d'acide cétylique, d'acide myristique, d'acide linoléique, d'acide octanoïque, d'acide oléique, ou également d'esters d'acide butyrique, d'acide propionique, d'acide acétique, ou aussi des esters d'acide hydroxylé tel que l'acide salicylique ou l'acide lactique, ou encore des esters d'acide cinnamique ou de dérivé d'acide cinnamique tel que l'acide férulique, ou des mélanges de ces esters.

L'ester utilisé selon l'invention peut être par exemple le mono- et dilaurate de dihydroxyacétone, le mono- et distéarate de dihydroxyacétone, le mono- et dipalmitate de dihydroxyacétone, le palmitate d'ascorbyle, le laurate d'ascorbyle, le myristate d'ascorbyle, le stéarate d'ascorbyle, le nicotinate d'ascorbyle, le palmitate de rétinyle, le propionate de rétinyle, l'acétate de rétinyle, le butyrate de rétinyle, l'octanoate de rétinyle, le laurate de rétinyle, l'oléate de rétinyle, le linoléate de rétinyle. Il peut s'agir aussi des mono- ou di-esters d'acide cinnamique ou de l'un de ses dérivés et d'acide ascorbique, et notamment ceux décrits dans le document FR-A-2715156, en particulier le 2-O-férulate d'ascorbyle et le 4'-acétoxyférulate de 2-O-(6-palmitoylascorbyle).

L'ester est utilisé dans le produit selon l'invention en une quantité allant de 0,1 à 50 % en poids, et de préférence de 0,5 à 10 % en poids par rapport au poids total de la composition quand il s'agit d'ester de vitamine, et de 5 à 30 % en poids par rapport au poids total de la composition quand il s'agit d'ester de cétones et notamment d'ester de dihydroxyacétone.

Selon une première variante de l'invention, on introduit la lipase et le précurseur dans une composition unique, qui est, de préférence, préparée juste avant l'emploi.

Selon une seconde variante, la lipase et le précurseur sont conditionnés de sorte à ne pas être en contact l'un avec l'autre, par exemple dans deux compositions différentes, qui peuvent être soit mélangées au moment de l'application soit appliquées de façon successive ou décalée dans le temps.

On peut par exemple disposer les compositions dans deux compartiments, qui sont en communication avec un conduit commun, d'où elles peuvent sortir en se mélangeant avant application sur la peau. De tels dispositifs de conditionnement en deux compartiments sont par exemple décrits dans les documents FR-A-2045559, FR-A-2105332, FR-A-2258319, FR-A-2293375, FR-A-2586913 ou FR-A-2643615.

On peut aussi réaliser l'une des compositions sous forme encapsulée et/ou sous forme de microcapsules ou de microgranulés immergés dans l'autre composition, les microcapsules ou les microgranulés étant écrasés au moment de l'application par frottement sur la peau, ce qui permet ainsi le mélange de la lipase et du précurseur et la libération de l'actif libre sur la peau.

Le produit selon l'invention peut être utilisé, selon l'ester d'actif qu'il contient, pour le traitement cosmétique et/ou dermatologique de la peau.

Le produit selon l'invention comporte avantageusement un milieu approprié pour une application topique, destiné aux domaines cosmétique et/ou dermatologique.

Aussi, l'invention a en outre pour objet l'utilisation du produit tel que défini ci-dessus pour préparer une pommade ou un onguent dermatologique destiné au traitement thérapeutique de la peau.

L'invention a encore pour objet l'utilisation du produit tel que défini ci-dessus pour le traitement cosmétique de la peau.

L'invention a encore pour objet un procédé de traitement cosmétique de la peau consistant à appliquer sur la peau de façon simultanée ou décalée une enzyme et au moins un précurseur d'un actif cosmétique ou dermatologique comportant au moins une fonction hydroxyle, caractérisé en ce que que l'enzyme est une lipase, en ce que l'actif cosmétique et/ou dermatologique est choisi dans le groupe comprenant les vitamines estérifiables et les cétones hydroxylées et en ce que le précurseur est un ester comportant au moins une fonction ester à chaîne linéaire ou ramifiée, saturée ou insaturée, ayant de 2 à 25 atomes de carbone.

Le milieu cosmétiquement et/ou dermatologiquement acceptable comprend généralement de l'eau, ou un mélange d'eau et de corps gras, ou un mélange de corps gras.

Comme corps gras utilisables dans l'invention, on peut citer les huiles minérales (vaseline, huile minérale), les huiles végétales et leurs dérivés hydrogénés, les huiles animales, les huiles de synthèse, les huiles siliconées (diméthicone, cyclométhicone) et les huiles fluorées. Comme autres corps gras, on peut encore citer les alcools gras, les acides gras et les cires.

En particulier, le produit peut se présenter sous forme de solutions aqueuses, alcooliques ou hydroalcooliques, de gels hydrophiles ou lipophiles, de microémulsions, d'émulsions eau-dans-huile ou huile-dans-eau ou eau-dans-huile-dans-eau ou huile-dans-eau-dans-huile ayant l'aspect d'une crème ou d'un gel, éventuellement aptes à mousser, sous forme d'aérosol, ou encore sous forme de dispersions vésiculaires contenant des lipides ioniques et/ou non ioniques. Ces formes galéniques sont préparées selon les méthodes usuelles des domaines considérés.

De façon connue, le milieu approprié pour une application topique selon l'invention peut contenir également des adjuvants habituels dans le domaine cosmétique ou dermatologique, tels que les gélifiants hydrophiles ou lipophiles, les tensioactifs, les actifs hydrophiles ou lipophiles, les conservateurs, les antioxydants, les solvants, les parfums, les charges, les filtres et les matières colorantes.

Les quantités des différents constituants du produit selon l'invention sont celles classiquement utilisées dans les domaines considérés.

Le produit selon l'invention peut constituer notamment des produits de protection, de traitement ou de soin pour le visage, pour le cou, pour les mains ou pour le corps, des produits de bronzage artificiel ou des produits pour les cheveux, et notamment pour le soin du cuir chevelu, par exemple sous forme de shampooings, de lotions traitantes, de crèmes ou de gels coiffants, de lotions ou de gels antichute.

Les exemples qui suivent sont donnés à titre illustratif afin de mieux faire comprendre l'invention. Les quantités indiquées sont des pourcentages en poids.

### Exemple 1 : Crème de soin pour la dépigmentation de la peau

| *Phase huileuse :* | |
|---|---|
| Triceteareth-4 phosphate/sodium C₁₄-C₁₇ alkyl sec sulfonate (Hostacerin CG vendu par la société Hoescht Celanese) (tensioactif) | 6 % |
| Vaseline | 2 % |
| Huile minérale | 4 % |
| Diméthicone | 3 % |
| Cyclométhicone | 3 % |
| Diméthicone copolyol (tensioactif) | 1 % |
| Triclosan (conservateur) | 0,1 % |
| Palmitate d'ascorbyle | 1 % |

| *Phase aqueuse* | |
|---|---|
| Propylène glycol (hydratant) | 2 % |
| PEG-20 (organoleptique) | 1 % |
| Lipolase 100 L | 1 % |
| Phénoxyéthanol (conservateur) | 0,4 % |
| Eau | qsp 100 % |

La lipolase 100 L est introduite dans la phase aqueuse sous forme encapsulée dans des microcapsules contenant aussi de l'atélocollagène et des glycosaminoglycannes.

Ces microcapsules sont immergées dans le reste des constituants après préparation de l'émulsion.

### Exemple 2 : Crème anti-rides

| *Phase huileuse* | |
|---|---|
| Triceteareth-4 phosphate/sodium C₁₄-C₁₇ alkyl sec sulfonate (Hostacerin CG vendu par la société Hoescht Celanese) (tensioactif) | 6 % |
| Vaseline | 2 % |
| Huile minérale | 4 % |
| Diméthicone | 3 % |
| Cyclométhicone | 3 % |
| Diméthicone copolyol (tensioactif) | 1 % |
| Triclosan (conservateur) | 0,1 % |
| Palmitate de rétinyle | 1 % |

| *Phase aqueuse* | |
|---|---|
| Propylène glycol (hydratant) | 2 % |
| PEG-20 (organoleptique) | 1 % |
| Lipolase 100 L | 1 % |
| Phénoxyéthanol (conservateur) | 0,4 % |
| Eau | qsp 100 % |

Le palmitate de rétinyle est introduit dans la composition sous forme de microsphères contenant aussi de l'atélocollagène et des glycosaminoglycannes.

Ces microsphères sont immergées dans le reste des constituants après préparation de l'émulsion.

### Exemple 3 : Crème solaire autobronzante

### A. Emulsion contenant l'ester de dihydroxyacétone :

| *Phase huileuse :* | |
|---|---|
| Steareth-2 (tensioactif) | 3 % |
| Steareth-21 (tensioactif) | 2 % |
| PPG-15 stearyl éther (tensioactif) | 29,5 % |
| Palmitate de dihydroxyacétone | 14,6 % |

| *Phase aqueuse :* | |
|---|---|
| Phénoxyéthanol (conservateur) | 0,5 % |
| Eau | qsp 100 % |

### B. Emulsion contenant la lipase :

| *Phase huileuse :* | |
|---|---|
| Steareth-2 (tensioactif) | 3 % |
| Steareth-21 (tensioactif) | 2 % |
| PPG-15 stearyl éther (tensioactif) | 29,5 % |

| *Phase aqueuse :* | |
|---|---|
| Phénoxyéthanol (conservateur) | 0,5 % |
| Lipase SP644 | 2 % |
| Eau | qsp 100 % |

Les émulsions A et B sont disposées dans deux compartiments séparés et mélangées au moment de l'application sur la peau.

Le produit obtenu donne, après application sur la peau, une coloration progressivement bronzée à la peau.

### Exemple 4 : Crème solaire autobronzante

### A. Emulsion contenant l'ester de dihydroxyacétone :

| *Phase huileuse :* | |
|---|---|
| Steareth-2 (tensioactif) | 3 % |
| Steareth-21 (tensioactif) | 2 % |
| PPG-15 stearyl éther (tensioactif) | 29,5 % |
| Laurate de dihydroxyacétone | 10 % |

| *Phase aqueuse :* | |
|---|---|
| Phénoxyéthanol (conservateur) | 0,5 % |
| Eau | qsp 100 % |

### B. Emulsion contenant la lipase :

| *Phase huileuse :* | |
|---|---|
| Steareth-2 (tensioactif) | 3 % |
| Steareth-21 (tensioactif) | 2 % |
| PPG-15 stearyl éther (tensioactif) | 29,5 % |

| *Phase aqueuse :* | |
|---|---|
| Phénoxyéthanol (conservateur) | 0,5 % |
| Lipase 100 L | 1 % |
| Eau | qsp 100 % |

Les émulsions sont diposées dans deux compartiments différents et sont mises en contact au moment de l'application.

### Exemple 5 : Crème anti-rides

| *Phase huileuse* | |
|---|---|
| Triceteareth-4 phosphate/sodium C₁₄-C₁₇ alkyl sec sulfonate (Hostacerin CG vendu par la société Hoescht Celanese) (tensioactif) | 5 % |
| Alcool stéarylique | 1 % |
| Vaseline | 2 % |
| Huile minérale | 4 % |
| Phényl triméthicone | 4 % |
| Cyclométhicone | 4 % |
| Diméthicone / Diméthiconol (tensioactif) | 2 % |
| Triclosan (conservateur) | 0,1 % |
| Palmitate de rétinyle | 0,6 % |

| *Phase aqueuse* | |
|---|---|
| Propylène glycol (hydratant) | 2 % |
| PEG-20 (organoleptique) | 1 % |
| Lipolase SP 644 | 0,5 % |
| Phénoxyéthanol (conservateur) | 0,2 % |
| Chlorphenesin | 0,2 % |
| Polyacrylamide/C13-C14 Isoparaffine/Laureth-7 (Sepigel 305 vendu par la société Seppic) (gélifiant) | 0,6 % |
| Eau | qsp 100 % |

Le palmitate de rétinyle est introduit dans la composition sous forme de microsphères contenant aussi de l'atélocollagène et du sodium chondroïtine sulfate.

Ces microsphères sont immergées dans le reste des constituants après préparation de l'émulsion.

### Exemple 6 : Crème de soin pour la dépigmentation de la peau

### A. Emulsion contenant l'ester de vitamine C :

| *Phase huileuse :* | |
|---|---|
| Steareth-2 (tensioactif) | 3 % |
| Steareth-21 (tensioactif) | 2 % |
| PPG-15 stearyl éther (tensioactif) | 9 % |
| Alcool cétylique | 2 % |
| Vaseline | 5 % |
| Triclosan (conservateur) | 0,2 % |
| Palmitate d'ascorbyle | 1 % |

| *Phase aqueuse :* | |
|---|---|
| Propylène glycol (hydratant) | 4 % |
| PEG-20 (organoleptique) | 5 % |
| Phénoxyéthanol (conservateur) | 0,5 % |
| Eau | qsp 100 % |

### B. Emulsion contenant la lipase :

| *Phase huileuse :* | |
|---|---|
| Steareth-2 (tensioactif) | 3 % |
| Steareth-21 (tensioactif) | 2 % |
| PPG-15 stearyl éther (tensioactif) | 9 % |
| Alcool cétylique | 2 % |
| Vaseline | 5 % |
| Triclosan (conservateur) | 0,2 % |

| *Phase aqueuse :* | |
|---|---|
| Propylène glycol (hydratant) | 4 % |
| PEG-20 (organoleptique) | 5 % |
| Phénoxyéthanol (conservateur) | 0,5 % |
| Lipase 100 L | 1 % |
| Eau | qsp 100 % |

Les émulsions A et B sont disposées dans deux compartiments séparés et mises en contact au moment de l'application sur la peau.

## Revendications

1. Produit pour application topique contenant une enzyme et au moins un précurseur d'un actif cosmétique et/ou dermatologique comportant au moins une fonction hydroxyle, caractérisé en ce que l'enzyme est une lipase, en ce que l'actif cosmétique et/ou dermatologique est choisi dans le groupe comprenant les vitamines estérifiables et les cétones hydroxylées et en ce que le précurseur est un ester comportant au moins une fonction ester à chaîne linéaire ou ramifiée, saturée ou insaturée, ayant de 2 à 25 atomes de carbone.

2. Produit selon la revendication 1, caractérisé en ce que la chaîne de la fonction ester est choisie parmi les radicaux acyle, benzoyle, alkylbenzoyle, acylbenzoyle, 2-hydroxyphénylacétyle, éventuellement substitués.

3. Produit selon la revendication 1 ou 2, caractérisé en ce que la chaîne de la fonction ester a de 12 à 18 atomes de carbone.

4. Produit selon l'une quelconque des revendications précédentes, caractérisé en ce que l'ester est choisi parmi les esters d'acide laurique, d'acide palmitique, d'acide stéarique, d'acide cétylique, d'acide myristique, d'acide propionique, d'acide linoléique, d'acide acétique, d'acide butyrique, d'acide octanoïque, d'acide oléique, d'acide cinnamique, d'acide férulique ou leurs mélanges.

5. Produit selon l'une quelconque des revendications précédentes, caractérisé en ce que l'actif cosmétique et/ou dermatologique est choisi dans le groupe comprenant le rétinol, l'acide ascorbique, la dihydroxyacétone.

6. Produit selon l'une quelconque des revendications précédentes, caractérisé en ce que l'ester est choisi dans le groupe comprenant le mono- et dilaurate de dihydroxyacétone, le mono- et distéarate de dihydroxyacétone, le mono- et dipalmitate de dihydroxyacétone, le palmitate d'ascorbyle, le laurate d'ascorbyle, le myristate d'ascorbyle, le stéarate d'ascorbyle, le nicotinate d'ascorbyle, le palmitate de rétinyle, le propionate de rétinyle, l'acétate de rétinyle, le butyrate de rétinyle, l'octanoate de rétinyle, le laurate de rétinyle, l'oléate de rétinyle, le linoléate de rétinyle, le 2-O-férulate d'ascorbyle, le 4'-acétoxyférulate de 2-O-(6-palmitoylascorbyle).

7. Produit selon l'une quelconque des revendications précédentes, caractérisé en ce que l'enzyme et le précurseur sont conditionnés de sorte à ne pas être en contact l'un avec l'autre.

8. Produit selon la revendication 7, caractérisé en ce que l'enzyme et le précurseur sont conditionnés dans des compartiments séparés.

9. Produit selon l'une quelconque des revendications précédentes, caractérisé en ce que l'enzyme et/ou le précurseur sont sous forme encapsulée.

10. Produit selon l'une quelconque des revendications précédentes, caractérisé en ce que l'enzyme et/ou le précurseur sont sous forme de microcapsules ou de microgranulés.

11. Produit selon l'une quelconque des revendications précédentes, caractérisé en ce que la lipase est choisie dans le groupe des enzymes de classification EC 3.1.1.3.

12. Produit selon l'une quelconque des revendications précédentes, caractérisé en ce que l'enzyme est présente en une quantité allant de 0,05 à 30 % en poids par rapport au poids total du produit.

13. Produit selon l'une quelconque des revendications précédentes, caractérisé en ce que l'enzyme est présente en une quantité allant de 0,1 à 10 % en poids par rapport au poids total du produit.

14. Produit selon l'une quelconque des revendications précédentes, caractérisé en ce que le précurseur est présent en une quantité allant de 0,1 à 50 % en poids par rapport au poids total du produit.

15. Produit selon l'une quelconque des revendications précédentes, caractérisé en ce que, lorsque le précurseur est un ester de vitamine, il est présent en une quantité allant de 0,5 à 10 % en poids par rapport au poids total du produit.

16. Produit selon l'une quelconque des revendications précédentes, caractérisé en ce que, lorsque le précurseur est un ester de cétone hydroxylée, il est présent en une quantité allant de 5 à 30 % en poids par rapport au poids total du produit.

17. Utilisation du produit selon l'une quelconque des revendications précédentes pour préparer une pommade ou un onguent dermatologique destiné au traitement thérapeutique de la peau.

18. Utilisation du produit selon l'une quelconque des revendications 1 à 16 pour le traitement cosmétique de la peau.

19. Utilisation du produit selon l'une quelconque des revendications 1 à 16 pour colorer la peau, l'actif cosmétique étant la dihydroxyacétone.

20. Utilisation du produit selon l'une quelconque des revendications 1 à 16 pour dépigmenter la peau, l'actif cosmétique étant l'acide ascorbique.

21. Procédé de traitement cosmétique de la peau consistant à appliquer sur la peau de façon simultanée ou décalée une enzyme et au moins un précurseur d'un actif cosmétique ou dermatologique comportant au moins une fonction hydroxyle, caractérisé en ce que l'enzyme est une lipase, en ce que l'actif cosmétique et/ou dermatologique est choisi dans le groupe comprenant les vitamines estérifiables et les cétones hydroxylées et en ce que le précurseur est un ester comportant au moins une fonction ester à chaîne linéaire ou ramifiée, saturée ou insaturée, ayant de 2 à 25 atomes de carbone.

## Claims

1. Product for topical application containing an enzyme and at least one precursor of a cosmetic and/or dermatological active ingredient containing at least one hydroxyl functional group, characterised in that the enzyme is a lipase, in that the cosmetic and/or dermatological active ingredient is chosen from the group comprising esterifiable vitamins and hydroxylated ketones and in that the precursor is an ester containing at least one ester functional group with a saturated or unsaturated, linear or branched chain having from 2 to 25 carbon atoms.

2. Product according to Claim 1, characterized in that the chain of the ester functional group is chosen from acyl, benzoyl, alkylbenzoyl, acylbenzoyl and 2-hydroxyphenylacetyl radicals, which are optionally substituted.

3. Product according to Claim 1 or 2, characterized in that the chain of the ester functional group has from 12 to 18 carbon atoms.

4. Product according to any one of the preceding claims, characterized in that the ester is chosen from esters of lauric acid, palmitic acid, stearic acid, cetylic acid, myristic acid, propionic acid, linoleic acid, acetic acid, butyric acid, octanoic acid, oleic acid cinnamic acid or ferulic acid, or their mixtures.

5. Product according to any one of the preceding claims, characterized in that the cosmetic and/or dermatological active ingredient is chosen from the group comprising retinol, ascorbic acid or dihydroxyacetone.

6. Product according to any one of the preceding claims, characterized in that the ester is chosen from the group comprising dihydroxyacetone mono- and dilaurate, dihydroxyacetone mono- and distearate, dihydroxyacetone mono- and dipalmitate, ascorbyl palmitate, ascorbyl laurate, ascorbyl myristate, ascorbyl stearate, ascorbyl nicotinate, retinyl palmitate, retinyl propionate, retinyl acetate, retinyl butyrate, retinyl octanoate, retinyl laurate, retinyl oleate, retinyl linoleate, ascorbyl 2-O-ferulate or 2-O-(6-palmitoylascorbyl) 4'-acetoxyferulate.

7. Product according to any one of the preceding claims, characterized in that the enzyme and the precursor are packaged so as not to be in contact with one another.

8. Product according to Claim 7, characterized in that the enzyme and the precursor are packaged in separate compartments.

9. Product according to any one of the preceding claims, characterized in that the enzyme and/or the precursor are in an encapsulated form.

10. Product according to any one of the preceding claims, characterized in that the enzyme and/or the precursor are in the form of microcapsules or of microgranules.

11. Product according to any one of the preceding claims, characterized in that the lipase is chosen from the group of enzymes of EC 3.1.1.3. classification.

12. Product according to any one of the preceding claims, characterized in that the enzyme is present in an amount ranging from 0.05 to 30 % by weight with respect to the total weight of the product.

13. Product according to any one of the preceding claims, characterized in that the enzyme is present in an amount ranging from 0.1 to 10 % by weight with respect to the total weight of the product.

14. Product according to any one of the preceding claims, characterized in that the precursor is present in an amount ranging from 0.1 to 50 % by weight with respect to the total weight of the product.

15. Product according to any one of the preceding claims, characterized in that, when the precursor is a vitamin ester, it is present in an amount ranging from 0.5 to 10 % by weight with respect to the total weight of the product.

16. Product according to any one of the preceding claims, characterized in that, when the precursor is a hydroxylated ketone ester, it is present in an amount ranging from 5 to 30 % by weight with respect to the total weight of the product.

17. Use of the product according to any one of the preceding claims for preparing a dermatological salve or ointment intended for the therapeutic treatment of the skin.

18. Use of the product according to any one of Claims 1 to 16 for the cosmetic treatment of the skin.

19. Use of the product according to any one of Claims 1 to 16 for colouring the skin, the cosmetic active ingredient being dihydroxyacetone.

20. Use of the product according to any one of Claims 1 to 16 for depigmenting the skin, the cosmetic active ingredient being ascorbic acid.

21. Process for the cosmetic treatment of the skin which consists in applying to the skin, simultaneously or with a time delay, an enzyme and at least one precursor of a cosmetic or dermatological active ingredient containing at least one hydroxyl functional group, characterized in that the enzyme is a lipase, in that the cosmetic and/or dermatological active ingredient is chosen from the group comprising esterifiable vitamins and hydroxylated ketones and in that the precursor is an ester containing at least one ester functional group with a saturated or unsaturated, linear or branched chain having from 2 to 25 carbon atoms.

## Patentansprüche

1. Produkt zur topischen Anwendung, das ein Enzym und mindestens einen Precursor eines kosmetischen und/oder dermatologischen Wirkstoffs, der mindestens eine Hydroxygruppe aufweist, enthält,
dadurch gekennzeichnet, daß
das Enzym eine Lipase ist, der kosmetische und/oder dermatologische Wirkstoff unter den veresterbaren Vitaminen und Ketonen mit Hydroxygruppen ausgewählt ist und der Precursor ein Ester ist, der mindestens eine geradkettige oder verzweigte, gesättigte oder ungesättigte Estergruppe mit 2 bis 25 Kohlenstoffatomen aufweist.

2. Produkt nach Anspruch 1, dadurch gekennzeichnet, daß die Kette der Estergruppe unter den Gruppen Acyl, Benzoyl, Alkylbenzoyl, Acylbenzoyl, 2-Hydroxyphenylacetyl ausgewählt ist, die gegebenenfalls substituiert sind.

3. Produkt nach Anspruch 1 oder 2, dadurch gekennzeichnet, daß die Kette der Estergruppe 12 bis 18 Kohlenstoffatome aufweist.

4. Produkt nach einem der vorhergehenden Ansprüche, dadurch gekennzeichnet, daß der Ester ausgewählt ist unter den Estern von Laurinsäure, Palmitinsäure, Stearinsäure, Cetylsäure, Myristinsäure, Propionsäure, Linolsäure, Essigsäure, Buttersäure, Octansäure, Ölsäure, Zimtsäure, Ferulasäure oder deren Gemischen.

5. Produkt nach einem der vorhergehenden Ansprüche, dadurch gekennzeichnet, daß der kosmetische und/oder dermatologische Wirkstoff unter Retinol, Ascorbinsäure und Dihydroxyaceton ausgewählt ist.

6. Produkt nach einem der vorhergehenden Ansprüche, dadurch gekennzeichnet, daß der Ester ausgewählt ist unter Dihydroxyacetonmono- und -dilaurat, Dihydroxyacetonmono- und -distearat, Dihydroxyacetonmono- und -dipalmitat, Ascorbylpalmitat, Ascorbyllaurat, Ascorbylmyristat, Ascorbylstearat, Ascorbylnicotinat, Retinylpalmitat, Retinylpropionat, Retinylacetat, Retinylbutyrat, Retinyloctanoat, Retinyllaurat, Retinyloleat, Retinyllinoleat, 2-O-Ascorbylferulat, 2-O-(6-Palmitoylascorbyl)-4'-acetoxyferulat.

7. Produkt nach einem der vorhergehenden Ansprüche, dadurch gekennzeichnet, daß das Enzym und der Precursor so verpackt sind, daß sie nicht miteinander in Kontakt stehen.

8. Produkt nach Anspruch 7, dadurch gekennzeichnet, daß das Enzym und der Precursor in getrennten Abteilungen verpackt sind.

9. Produkt nach einem der vorhergehenden Ansprüche, dadurch gekennzeichnet, daß das Enzym und/oder der Precursor in eingekapselter Form vorliegen.

10. Produkt nach einem der vorhergehenden Ansprüche, dadurch gekennzeichnet, daß das Enzym und/oder der Precursor in Form von Mikrokapseln oder Mikrogranulat vorliegen.

11. Produkt nach einem der vorhergehenden Ansprüche, dadurch gekennzeichnet, daß die Lipase unter den Enzymen der Klassifikation EG 3.1.1.3. ausgewählt ist.

12. Produkt nach einem der vorhergehenden Ansprüche, dadurch gekennzeichnet, daß das Enzym in einem Anteil im Bereich von 0,05 bis 30 Gew.-%, bezogen auf das Gesamtgewicht des Produkts, vorliegt.

13. Produkt nach einem der vorhergehenden Ansprüche, dadurch gekennzeichnet, daß das Enzym in einem Anteil im Bereich von 0,1 bis 10 Gew.-%, bezogen auf das Gesamtgewicht des Produkts, vorliegt.

14. Produkt nach einem der vorhergehenden Ansprüche, dadurch gekennzeichnet, daß der Precursor in einem Anteil im Bereich von 0,1 bis 50 Gew.-%, bezogen auf das Gesamtgewicht des Produkts, vorliegt.

15. Produkt nach einem der vorhergehenden Ansprüche, dadurch gekennzeichnet, daß, wenn der Precursor ein Vitamin ist, er in einem Anteil im Bereich von 0,5 bis 10 Gew.-%, bezogen auf das Gesamtgewicht des Produkts, vorliegt.

16. Produkt nach einem der vorhergehenden Ansprüche, dadurch gekennzeichnet, daß, wenn der Precursor ein Ester eines Ketons mit Hydroxygruppen ist, er in einem Anteil im Bereich von 5 bis 30 Gew.-%, bezogen auf das Gesamtgewicht des Produkts, vorliegt.

17. Verwendung des Produkts nach einem der vorhergehenden Ansprüche zur Herstellung einer Salbe oder dermatologischen Salbe, die zur therapeutischen Behandlung der Haut bestimmt ist.

18. Verwendung des Produkts nach einem der Ansprüche 1 bis 16 zur kosmetischen Behandlung der Haut.

19. Verwendung des Produkts nach einem der Ansprüche 1 bis 16 zur Färbung der Haut, wobei der kosmetische Wirkstoff Dihydroxyaceton ist.

20. Verwendung des Produkts nach einem der Ansprüche 1 bis 16 zur Depigmentierung der Haut, wobei der kosmetische Wirkstoff Ascorbinsäure ist.

21. Verfahren zur kosmetischen Behandlung der Haut, das darin besteht, auf die Haut gleichzeitig oder zeitlich voneinander getrennt ein Enzym und mindestens einen Precursor eines kosmetischen oder dermatologischen Wirkstoff, der mindestens eine Hydroxygruppe aufweist, aufzutragen, dadurch gekennzeichnet, daß das Enzym eine Lipase ist, der kosmetische und/oder dermatologische Wirkstoff unter den veresterbaren Vitaminen und Ketonen mit Hydroxygruppen ausgewählt ist und der Precursor ein Ester ist, der mindestens eine lineare oder verzweigte, gesättigte oder ungesättigte Esterkette mit 2 bis 25 Kohlenstoffatomen enthält.
